# EUROPÄISCHE PATENTSCHRIFT

(11) **EP 1 829 548 B1**
(45) Veröffentlichungstag und Bekanntmachung des Hinweises auf die Patenterteilung: **04.02.2009**
(21) Anmeldenummer: 06004114.2
(22) Anmeldetag: 01.03.2006
(51) Int. Cl.: A61K 36/68, A61K 36/185, A61P 1/02, A61P 11/04

(54) **Verwendung von Pelargonium Extrakten in Kombination mit Plantago zur Herstellung eines Medikaments zur Behandlung der Rachenentzündung**
Use of Pelargonium extracts in combination with Plantago for the preparation of a medicament for the treatment of pharyngitis
Utilisation d'extraits de Pelargonium en association avec de Plantago pour la préparation d'un médicament pour le traitement de la pharyngite

(43) Veröffentlichungstag der Anmeldung: 05.09.2007
(60) Teilanmeldung: 08011644.5
(73) Patentinhaber: Divapharma Chur AG, 7006 Chur (CH)
(72) Erfinder: Schneider, Ernst, Dr., 84163 Marklkofen (DE); Ploch, Michael, Dr., 16565 Lehnitz (DE)
(74) Vertreter: Gesthuysen, von Rohr & Eggert

(56) Entgegenhaltungen:
- EP-A- 0 870 507
- GB-A- 2 093 696
- "Husten und Bronchitis" ZEITSCHRIFT FÜR PHYTOTHERAPIE, Bd. 26, Nr. 6, 2005, Seiten 282-285, XP008067465
- "Extract of Pelargonium sidoides: South African Herbal Remedy Successfully Treats Acute Bronchitis and Tonsillopharyngitis" JOURNAL OF THE AMERICAN BOTANICAL COUNCIL, [Online] Bd. 63, 2004, XP002394133 Gefunden im Internet: URL:http://www.herbalgram.com/bodywise/her balgram/articleview.asp?a=2703&p=Y> [gefunden am 2006-08-08]
- GALVEZ PERALTA, M.: "Estudios de los componentes químicos de Plantago spp. como posibles agentes antitumorales"[Online] 2004, Seiten 3-41, XP002394134 Gefunden im Internet: URL:http://fondosdigitales.us.es/public_th esis/394/9198.pdf> [gefunden am 2006-08-08]
- SAMUELSEN, A.B.: "The traditional uses, chemical constituents and biological activities of Plantago major L." JOURNAL OF ETHNOPHARMACOLOGY, Bd. 71, Nr. 1-2, Juli 2000 (2000-07), Seiten 1-21, XP002933658

## Beschreibung

Die vorliegende Erfindung betrifft die Verwendung von Extrakten aus Pelargonium sidoides und/oder Pelargonium reniforme in Kombination mit Plantago major und/oder Plantago lanceolata zur topischen Prophylaxe oder Behandlung von Entzündungen des Mund- und Rachenraums in Form von Lutschpastillen.

Dem Gebiet der Phytopharmaka kommt eine wachsende Bedeutung zu. Dabei sind bereits etliche Pflanzen oder Pflanzenextrakte bekannt, denen bestimmte vorteilhafte Wirkungen zugeschrieben werden. Zu diesen zählen *Pelargonium sidoides* und Spitzwegerich.

### Pelargonium sidoides

Die Herkunft dieser auch als Kap-Pelargonie bezeichneten Pflanze ist Südafrika, von wo auch eine Reihe wissenschaftlicher Belege zur Ethnobotanik vorliegen (Van Wyk 1997, 2000, 2004). In Südafrika werden neben P. sidoides viele ganz ähnlich aussehende Pelargonium-Arten als traditionelle Arzneipflanzen und auch als Lebensmittel, sowie für andere Zwecke (Parfüm, Gerbstoff) verwendet. Wegen der bekannten Ungiftigkeit werden Blätter und Stiel aller Arten gerne als "bush food snack" verzehrt, aber auch die Wurzeln als Gemüse für die Vorratshaltung geerntet.

Diese Pflanze ist bislang nur in dem Arzneimittel "Umckaloabo^{®} Lösung zum Einnehmen" mit der Zusammensetzung: 100 g, enthaltend einen wässrig-ethanolischen Auszug aus den Wurzeln von *Pelargonium reniforme*/*sidoides* (1:10) 80 g, sowie Glycerol, enthalten.

### Plantago: Spitzwegerich und Breitwegerich

Die volkstümliche Anwendung des Spitzwegerich und Breitwegerich war bereits im Altertum gebräuchlich und er wurde auch in den alten Kräuterbüchern hoch geschätzt (Marchesan 1998). Auffällig ist die weltweite Kenntnis der Eigenschaften und Anwendungen dieser Pflanze bei vielen Völkern. Dies könnte ein Hinweis sein, dass die positiven Effekte eindeutig erfahrbar sind (Paper 1999).

Spitzwegerich wird bei uns auch als Wildgemüse angesehen (Helm 1988) und wie die nahe Verwandte, auch kommerziell gehandelte mediterrane Art *P*. *coronopus* (Liebster 1990) zu Salaten und Gemüse verwendet.

Im Verzeichnis der zulässigen Bestandteile von Kosmetika gemäß der EU-Richtlinie (INCI Liste) wird Spitzwegerich als "botanical" aufgeführt. Mit dieser funktionellen Eigenschaft werden Zusätze bezeichnet, mit denen spezifische Rezeptureigenschaften erreicht werden sollen (INCI 2005).

Die Patentschrift EP-A-0 870 507 (FARMO-NAT LTD., 14. Oktober 1998) offenbart eine antimikrobielle Wirkstoffkombination, die Geranienöl in Kombination mit *Plantago major* enthält, für die topische Behandlung von Infektionskrankheiten. Das Dokument "Husten und Bronchitis" (ZEITSCHRIFT FÜR PHYTOTHERAPIE, Bd. 26, Nr. 6, 2005, Seiten 282-285) offenbart die Verwendung von *Pelargonium sidoides-* und/oder *Pelargonium reniforme*-Extrakten für die systemische Behandlung von Entzündungen des Mund- und Rachenraums sowie die Verwendung von *Plantago lanceolata* für die topische Behandlung von Entzündungen des Mund- und Rachenraums. Das Dokument SAMUELSEN, A.B.: "The traditional uses, chemical constituents and biological activities of Plantago major L." (JOURNAL OF ETHNOPHARMACOLOGY, Bd. 71, Nr. 1-2, Juli 2000, Seiten 1-21) offenbart die Verwendung von *Plantago major* für die topische Behandlung von Entzündungen des Mund- und Rachenraums.

Beide oben genannten Pflanzen bzw. ihre Extrakte sind insbesondere für ihre systemischen Wirkungen bekannt und werden als solche eingesetzt. Typischerweise werden solche systemischen Wirkungen insbesondere durch Rezeptoren im Körper des Betroffenen vermittelt. Solche Rezeptorvermittelten Wirkungen können wie folgt gekennzeichnet werden:

### Rezeptor

Eine rezeptorvermittelte Wirkung erfordert einen spezifisch auf die Wirksubstanz abgestimmten Rezeptor (Schlüssel-Schloss-Prinzip). Hierbei kommt es zu Verdrängungseffekten zwischen der Testsubstanz (z.B. β-Blocker) und dem körpereigenen Liganden (z.B. Adrenalin), woraus eine typische Dosisabhängigkeit resultiert. Bindet eine Substanz an einen Rezeptor, wird dadurch eine physiologisch vorgegebene Reaktion oder Reaktionsfolge ausgelöst, die als typischer Effekt (Wirkung) in pharmakologischen Experimenten messbar ist.

### Dosis-Wirkungs-Kurve

Wertet man Daten aus pharmakologischen Experimenten graphisch so aus, dass auf der Abszisse der dekadische Logarithmus der Dosis und auf der Ordinate linear die Wirkung aufgetragen wird, erhält man typische sigmoide Kurven.

Solche Kurven treten bei physikalischen Prozessen nicht auf.

Demgegenüber liegen typischen topischen Behandlungsformen keine Rezeptorwechselwirkungen zugrunde; vielmehr sind topische Behandlungen auf rein physikalische oder physikochemische Prozesse zurückzuführen.

Es sind etliche Krankheitsbilder bekannt, bei denen eine topische Anwendung notwendig sein kann. Solche Krankheitsbilder oder Störungen sind z.B. entzündliche Reaktionen, wie z.B. im Hals-, Rachen- oder Mundraum, Entzündungen der Haut, wie Neurodermitis, Gürtelrose oder Windeldermatitis oder auch Magenentzündungen. Insbesondere der Hautentzündung, besonders der Neurodermitis kommt eine wachsende Bedeutung im Hinblick auf die Tatsache zu, dass die Zahl der Neurodermitisfälle, insbesondere in den zivilisierten Ländern drastisch angestiegen ist. Ferner kommt auch den Entzündungen des Mund- und Rachenraums eine große Bedeutung zu, da diese nach wie vor extrem häufig anzutreffen sind und es weiterhin schwierig bleibt, solche Entzündungen sowohl von Grund auf als auch symptomatisch zu behandeln.

Es ist daher eine Aufgabe der vorliegenden Erfindung, eine neue Verwendung von bekannten Phytopharmaka bereitzustellen, wobei diese Verwendung zur Behandlung oder Prophylaxe von Entzündungen des Mund- und Rachenraums geeignet ist.

Diese Aufgabe wird durch die in Anspruch 1 angegebene Verwendung gelöst, nämlich durch die Verwendung von Pelargonium sidoides und/oder Pelargonium reniforme in Kombination mit Plantago major und/oder Plantago lanceolata für die topische Prophylaxe und/oder Behandlung von Entzündungen des Mund- und Rachenraums.

Bevorzugte Ausführungformen sind in den Unteransprüchen angegeben.

Im folgenden wird in der Beschreibung "Spitzwegerich" als Synonym für alle Plantago-Arten verwendet.

Da die Verwendung auf eine topische Prophylaxe oder Behandlung abzielt, sollte die Darreichungsform eine Lutschpastille, eine Lutschtablette, eine Kautablette, eine Kapsel, ein Schaum, ein Mund- oder Rachenspray, ein Kaugummi, oder eine Gurgellösung sein, insbesondere in den Fällen, in denen die zu behandelnde Erkrankung eine Erkrankung des Mund-und Rachenraumes ist. Dabei wird durch z.B. das Lutschen der Pastille oder der Tablette erreicht, dass sich die Wirkstoffe sehr langsam im Mund- und Rachenbereich auflösen und die betroffenen Zellen nur in geringer Konzentration erreichen. Dazu trägt auch bei, dass die bevorzugte Konzentration der Wirkstoffe in der Tablette, insbesondere des Pelargonium-Extrakts gemäß den bevorzugten Ausführungsformen deutlich niedriger gewählt ist als diejenige in konventionellen Pelargonium-Präparaten. Dadurch wird der Effekt erreicht, dass die z.B. entzündeten Proteine im Bereich des Mund- und Rachenraums lediglich topisch von dem Pelargonium-Extrakt und unter Umständen dem Spitzwegerich-Extrakt erreicht werden, und sich dabei verbrauchen, ohne dass eine systemische Aufnahme der Wirkstoffe erfolgt. Auf diese Weise wird in besonders bevorzugter Weise erreicht, dass die, wenn auch geringen, Pelargonium oder Spitzwegerich zugeschriebenen systemischen Nebenwirkungen im gegenwärtigen Fall einer topischen Anwendung nicht auftreten sollten.

Es ist außerdem möglich, der gewählten Darreichungsform, z.B. insbesondere wenn es sich um eine Lutschpastille oder ein Kaugummi handelt, die Freisetzung verzögernde Stoffe beizufügen, die auf dem Gebiet bekannt sind und die dazu führen, dass die Wirkstoffe in besonders geringer Konzentration und über einen längeren Zeitraum hinweg den betroffenen Zonen topisch zugeführt werden, ohne dass es zu einer substantiellen systemischen Aufnahme kommt. Insbesondere sind die Darreichungsformen gemäß der vorliegenden Erfindung so formuliert, dass zunächst die Wirkung des Pelargonium-Bestandteils überwiegt worauffolgend die Wirkung des Spitzwegerichs überwiegt. Ein solches Freisetzungsmuster kann eine auf dem Fachmann bekannte Weise verwirklicht werden. Zum Beispiel ist es möglich, Pelargonium und Spitzwegerich mit unterschiedlichen Trägern zu formulieren, die ihre Wirkstoffe in unterschiedlich schneller Weise freisetzen. Zum Beispiel kann Pelargonium mit einem leicht löslichen Zuckeralkohol formuliert werden, während Spitzwegerich mit einem schwerer löslichen Zucker-Alkohol vermischt wird. Auf diese Weise ergibt sich ein Freisetzungsmuster, wobei zunächst die Wirkung des Pelargonium-Bestandteils überwiegt. Dadurch können die Gerbstoffe des Pelargonium-Bestandteils zunächst die Proteine in der Mundschleimhaut vernetzen, woraufhin die Wirkstoffe des Spitzwegerichs eine schützende Schleimschicht über dieselbe legen können.

Eine weitere Möglichkeit, eine verzögerte Freisetzung zu erreichen, ist z.B. die Formulierung in Liposomen, die ebenfalls dem Fachmann auf dem Gebiet bekannt ist.

Noch eine weitere Möglichkeit ist die Formulierung in einer zweischichtigen Lutschpastille, die derartig aufgebaut ist, dass zunächst der Pelargonium-Bestandteil und darauffolgend oder zeitlich überlappend der Spitzwegerich-Bestandteil freigesetzt wird.

Es sind auch Formulierungen denkbar, bei denen ein Bestandteil eine Matrix bildet, in der der weitere Bestandteil eingeschlossen ist. Kombinationen der obigen Möglichkeiten sind selbstverständlich ebenfalls möglich und denkbar.

Die Erfindung umfasst hier ausdrücklich solche Ausführungsformen, in denen die Bestandteile Pelargonium sidoides und/oder Pelargonium reniforme und Plantago major und/oder Plantago lanceolata zeitlich voneinander abgesetzt, d.h. nacheinander freigesetzt werden, als auch solche, in denen Plantago major und/oder Plantago lanceolata und Pelargonium sidoides und/oder Pelargonium reniforme zunächst gemeinsam, unter Umständen in einem überwiegenden Anteil Pelargonium sidoides und/oder Pelargonium reniforme, freigesetzt werden, worauf eine Phase folgt, in der überwiegend oder ausschließlich Plantago major und/oder Plantago lanceolata freigesetzt wird.

Wenn es sich bei der zu behandelnden Erkrankung um eine Magen- oder Darm-Entzündung handelt, werden insbesondere Pastillen oder Tabletten als auch z.B. Kaugummis und Weich- oder Hart- Gelatine-Kapseln bevorzugt, die wiederum in ihrer Dosierung ähnlich den bevorzugten Dosierungsformen wie unten beschrieben für Lutschpastillen einen verhältnismäßig niedrigen Wirkstoffgehalt im Vergleich zu dem konventionellen Wirkstoffgehalt für Pelargonium und/oder Spitzwegerich enthalten sowie wiederum bevorzugt Stoffe, die die Freisetzung der Wirkstoffe verzögern und die auf dem Gebiet dem Fachmann bekannt sind. Dabei sind diese Rezepturen so gewählt, dass sie je nach Erkrankung im Magen bzw. Darm freigesetzt werden. Für eine Freisetzung im Darm sind dem Fachmann z.B. etliche magensaftresistente Beschichtungen bekannt, wie z.B. Eudragit.

Handelt es sich um die Behandlung einer Hautentzündung, wie z.B. einer Neurodermitis, einer Gürtelrose oder einer Windeldermatitis oder um die Prophylaxe derselben, sind die bevorzugten Darreichungsformen z.B. eine Creme, eine Salbe oder ein Gel. Die Creme, die Salbe oder das Gel können weitere übliche Zusatz- und/oder Hilfsstoffe und Creme-, Salben- oder Gel-Grundlagen enthalten, die wiederum dem Fachmann auf dem Gebiet wohl bekannt sind.

Die Verwendung erfolgt gemäß der vorliegenden Erfindung in Kombination mit Spitzwegerich, nämlich Plantago lanceolata, das in Form eines Trockenextrakts verwendet werden kann. Eine weitere bevorzugte Verwendung, insbesondere der Kombination von einem wässrig-ethanolischen Extrakt von *Pelargonium sidoides* und/oder *reniforme* mit Trockenextrakt vom Spitzwegerich erfolgt in derselben Darreichungsform, und insbesondere bevorzugt in Form einer Lutschpastille.

Diese bevorzugte Ausführungsform wird für die Behandlung von Entzündungen im Mund- und Rachenraum verwendet.

Bei den zu behandelnden Störungen oder Erkrankungen oder den Störungen oder Erkrankungen, denen vorgebeugt werden soll, handelt es sich insbesondere um eine Mund- oder Rachenentzündung, eine Magen- oder Darm-Entzündung oder eine Entzündung der Haut, wobei die Haut-Entzündung z.B. eine Neurodermitis, eine Gürtelrose oder eine Windeldermatitis sein kann. Bei der Darmentzündung handelt es sich insbesondere um Morbus Crohn oder eine Colitis ulcerosa.

Die Funktionsweise der Lutschpastille mit der gerbstoffhaltigen Pflanze Pelargonium und der Schleimhaltigen Pflanze Spitzwegerich ergibt sich besonders vorteilhaft aus der Form einer Kombinations-Zubereitung.

Dabei werden zunächst die aus der Pastille freigesetzten Gerbstoffe von *Pelargonium sidoides* Proteine der entzündeten Schleimhaut in Mund und Rachen vernetzen. Infektionen wird so vorgebeugt bzw. vorhandene Infektionen werden abgeschwächt. Die aus der Pastille herausgelösten Schleimstoffe des Spitzwegerich umgeben in der Folge die Schleimhäute mit einem schützenden Film. Unterstützt wird diese Schleimwirkung durch die Eigenschaft des optional vorhandenen Arabisches Gummi, mit Wasser zu einem schleimartigen Hydrocolloid aufzuquellen.

Diese Funktion wird durch die Darreichungsform der Pastillen erreicht, die so gelutscht werden, dass sie möglichst lange im Mundraum verbleiben.

Durch die bereits oben erwähnten bevorzugten Formulierungen, die so aufgebaut werden, dass die beiden Hauptwirkbestandteile in verzögerter Abfolge freigesetzt werden, kann die Wirkung jedoch sogar dann erreicht werden, wenn der Anwender die Pastille oder Tabelle nicht lutscht sondern kaut.

Es war besonders überraschend im gegenwärtigen Zusammenhang, dass es überhaupt möglich ist die beiden Wirkbestandteile Pelargonium und Spitzwegerich zu kombinieren. Es ist seit Jahrhunderten bekannt, dass es in der Regel nicht ratsam ist, Gerbstoffe und Schleimstoffe zu kombinieren. Da Pelargonium insbesondere Gerbstoffe und Spitzwegerich insbesondere Schleimstoffe umfasst, würde eine Kombination dieser beiden Wirkbestandteile basierend auf dem Wissen auf dem Gebiet von vornherein ausgeschlossen werden. Zum Beispiel ist es Apothekern seit langem bekannt, dass die beiden Bestandteile nicht zusammen in Lösung aufbewahrt werden können, da die Gerbstoffe die Schleimstoffe vernetzen würden und somit in der Lösung ausfallen würden.

Überraschend ist die spezifische Kombination aus Pelargonium sidoides und/oder Pelargonium reniforme und Plantago major und/oder Plantago lanceolata dennoch möglich und führt zu den oben dargestellten besonders vorteilhaften Wirkungen der vorliegenden Erfindung. Dabei vernetzen die Gerbstoffe zunächst die entzündeten Proteine der (Schleim-)haut woraufhin sich die Schleimstoffe des Spitzwergerichkrauts an die Gerbstoffe haften, wobei die Vernetzung der Schleimstoffe durch u. U. noch nicht vollständig abgefangene Gerbstoffe die vorteilhafte Wirkung aufweist, dass die Schleimstoffe besser an dem entzündeten Gewebe anhaften und dadurch in besonders vorteilhafter Weise ihre schützende Funktion ausüben können. Allerdings sind die Gerbstoffe in aller Regel so niedrig dosiert, dass ein wesentlicher Bestandteil bereits durch die Vernetzung der entzündeten Proteine verbraucht sein wird; etwaige noch vorhandene Rest-Gerbstoffe würden dann die Schleimstoffe vernetzen. Auf diese Weise wird sicher gestellt, dass keinerlei Gerbstoffe in u.U. nicht erwünschter Weise bei einer Behandlung des Rachenraums in den Magen oder Darmtrakt gelangt oder gar systemische Wirkungen entfaltet.

Um der oben erwähnten Hautentzündung, insbesondere einer Neurodermitis vorzubeugen, ist es außerdem möglich, die Kombination aus Pelargonium und Spitzwegerich-Extrakten in einen Bade- oder Waschzusatz zu formulieren, wobei diese Bade- oder Waschzusätze weiter dem Fachmann bekannte Hilfsstoffe umfassen können.

Sowohl die Formulierung in eine Creme, Salbe oder ein Gel wie auch die Formulierung in einen Bade- oder Waschzusatz hat den Effekt, dass Hautentzündungen topisch behandelt werden können; dabei wird wiederum die Wirkung dadurch erreicht, das die in Pelargonium sidoides bzw. reniforme-Extrakt enthaltenen Gerbstoffe Proteine der entzündeten Haut vernetzen und die Entzündung dadurch abschwächen können bzw. einer solchen vorbeugen. Die weiteren Schleimstoffe, die durch den Spitzwegerichextrakt bereit gestellt werden umgeben darauf folgend die Scheimhäute mit einem schützenden Film.

Dadurch wird auf topische Weise ohne Eingriff in das systemische Bild eines betroffenen Subjekts eine sehr vorteilhafte Behandlung von entzündlichen Erkrankungen möglich.

Um die oben beschriebenen Wirkungen weiter zu unterstützen sollte eine Lutschpastille, die eine Kombination aus Pelargonium-Extrakt und Spitzwegerich-Extrakt umfasst, besonders vorteilhaft eine zweischichtige Lutschpastille sein, wobei eine innere Kernschicht durch den Spitzwegerichbestandteil gebildet wird und eine äußere Beschichtung durch den Pelargonium-Bestandteil gebildet wird.

Auf diese Weise wird die oben erwähnte Wirkung besonders bevorzugt erreicht, indem nämlich zunächst die äussere Beschichtigung, die durch den Pelargonium-Bestandteil gebildet wird, gelutscht wird und dadurch die Proteine der entzündeten Schleimhaut im Mundrachen vernetzt, während nach der Vernetzung durch den Pelargonium-Bestandteil die innere Kernschicht, die durch den Spitzwegerich-Bestandteil gebildet wird, gelutscht wird und die bereits vernetzten Proteine dann mit einem schützendem Film überzogen werden.

Zusätzlich zu den bekannten Zusatzstoffen auf dem Gebiet der pharmazeutischen Technologie für die obenerwähnten bevorzugten und weitere Formulierungs- und Darreichungsformen werden im gegenwärtigen Kontext besonders die folgenden Zusatzstoffe bevorzugt: Arabisches Gummi, Maltitol, Sorbitol, Xylitol, Saccharose, Isomalt, wassserfreie Citronensäure, Acesulfam Kalium, dünnflüssiges Paraffin, gebleichtes Wachs, Wasser, Cellulose-Derivate, Zuckeralkohole, Eudragit und/oder Aromastoffe, insbesondere sind diese für die Formulierung einer Lutschpastille geeignet.

Bei den Aromastoffen handelt es sich bevorzugt um Geraniumöl und/oder Menthol. Besonders bevorzugt umfasst eine Lutschpastille, die eine Kombination aus Pelargonium-Extrakt und Spitzwegerich-Extrakt umfasst 80 bis 200 mg Pelargonium Urtinktur und 15 bis 40 mg Spitzwegerich-Extrakt. In einer weiteren besonders bevorzugten Ausführungsform umfasst eine solche Lutschpastille 100,00 mg Pelargonium Urtinktur und 25,00 mg Spitzwegerich-Extrakt. Im Hinblick auf den Spitzwegerichextrakt handelt es sich jeweils um einen 90% nativen Extrakt. Besonders bevorzugt wird die folgende Zusammensetzung für eine solche Lutschpastille:

| | |
|---|---|
| Pelargonium-Urtinktur: | 80 - 200 mg |
| Spitzwegerich-Trockenextrakt: | 15 - 40 mg |
| Arabisches Gummi: | 300 - 500 mg |
| Maltitollösung: | 400 - 600 mg |
| Sorbitollösung 70% (nicht kristallisierend): | 100 - 200 mg |
| Wasserfreie Citronensäure: | 4 - 9 mg |
| Menthol: | 0 - 100 mg |
| Acesulfam Kalium: | 0,3 - 1 mg |
| Geraniumöl, natürlich: | 0 - 0,1 mg |
| Dünnflüssiges Paraffin: | 0 - 2 mg |
| Gebleichtes Wachs: | 0 - 0,1 mg |
| Prozesswasser: | q.s. |

Das bevorzugt Verhältnis von Pelargoniumtrockensubstanz zu Spitzwegerich-Trockenextrakt beträgt 1:50 bis 1:8, besonders bevorzugt 1:40 bis 1:20, noch bevorzugter ca. 1:25 (G/G).

Noch bevorzugter ist die folgende Ausführungsform für eine solche Pastille:

| | |
|---|---|
| Pelargonium-Urtinktur: | 100 mg |
| Spitzwegerich-Trockenextrakt: | 25 mg |
| Arabisches Gummi: | 412 mg |
| Maltitollösung: | 553 mg |
| Sorbitollösung 70% (nicht kristallisierend): | 143 mg |
| Wasserfreie Citronensäure: | 6,50 mg |
| Menthol: | 0,80 mg |
| Acesulfam Kalium: | 0,5 mg |
| Geraniumöl, natürlich: | 0,05 mg |
| Dünnflüssiges Paraffin: | 1,30 mg |
| Gebleichtes Wachs: | 0,7 mg |
| Prozesswasser: | q.s., bei einem Pastillengewicht von 1.000,00 mg. |

In einer Darreichungsform für Pastillen zum Lutschen ist daher die folgende Zusammensetzung denkbar:
1 Pastille enthält
100,0 mg Pelargonium-Urtinktur
   (wässrig-ethanolischer Auszug aus der Wurzel von *Pelargonium sidoides*)
25,0 mg Spitzwegerich-Trockenextrakt

### Weitere Bestandteile:

Arabisches Gummi, Maltitol, Sorbitol, wasserfreie Citronensäure, Acesulfam Kalium, Dünnflüssiges Paraffin, gebleichtes Wachs, Wasser, Aroma (Geraniumöl, Menthol), unterschiedlich lösliche Zuckeralkohole.

Eine bevorzugte Zweckbestimmung wäre eine Anwendung zur Linderung der Beschwerden bei leichten und mittelschweren Erkrankungen des Mund- und Rachenraumes.

Diese Halspastillen sind zur Anwendung in der Mundhöhle bestimmt.

Die Lutschpastillen sollen so gelutscht werden, dass sie möglichst lange (ca. 10 Minuten) im Mundraum verbleiben.

Die Anwendung bei Kindern ab 6 Jahren und Erwachsenen sollte zu Beginn stündlich, danach bei Bedarf alle 2-3 Stunden mit jeweils einer Pastille erfolgen.

Wie bereits oben erklärt lässt sich die Funktionweise eines Kombinationspräparates in Form einer Lutschpastille wie folgt erklären:

Die Funktionsweise der Lutschpastille mit der gerbstoffhaltigen Pflanze Pelargonium und der Schleimhaltigen Pflanze Spitzwegerich ergibt sich besonders vorteilhaft aus der Form einer Kombinations-Zubereitung.

Dabei werden zunächst die aus der Pastille freigesetzten Gerbstoffe von *Pelargonium sidoides* Proteine der entzündeten Schleimhaut in Mund und Rachen vernetzen. Infektionen wird so vorgebeugt bzw. vorhandene Infektionen werden abgeschwächt. Die aus der Pastille herausgelösten Schleimstoffe des Spitzwegerich umgeben in der Folge die Schleimhäute mit einem schützenden Film. Unterstützt wird diese Schleimwirkung durch die Eigenschaft des optional vorhandenen Arabisches Gummi, mit Wasser zu einem schleimartigen Hydrocolloid aufzuquellen.

Diese Funktion wird durch die Darreichungsform der Pastillen erreicht, die so gelutscht werden, dass sie möglichst lange im Mundraum verbleiben.

Eine weitere denkbare Darreichungsform wäre eine Formulierung, bei der Pelargonium in höherer Dosierung als der oben genannten enthalten ist. Diese höhere Dosierung hätte den Vorteil, dass additiv zu der topischen vorteilhaften Wirkung, die Pelargonium-Gerbstoffe auch direkt an der Stelle des immunologischen Zentrums im Rachen, nämlich dem Waldeyer'schen Rachenring wirken könnten. Hier haben Gerbstoffe eine das Immunsystem unterstützende Wirkung. Besonders überraschend wurde gemäß der vorliegenden Erfindung festgestellt, das die Kombination aus Pelargonium und Spitzwegerich in dem Fall, in dem Pelargonium hoch dosiert verwendet wird, eine synergistische Wirkung aufweist, da die hoch verzweigten Polysaccharide des Spitzwegerichkrauts ebenfalls immunkompetente Zellen anregen. Ferner kann sich durch die vorherige Vernetzung der Gerbstoffe die Schleimschicht der Schleimstoffe des Spitzwegerichs nicht nur lose anlagern sondern sich fest vernetzt anhaften. Diese Wirkung kann auch bei niedrig dosierten Gerbstoffen in synergistischer Weise die Wirkung der Lutschpastille verbessern.

Weiterhin ist es eine Ausführungsform der vorliegenden Erfindung denkbar, in der die genannten Wirkstoffe Pelargonium sidoides und/oder Pelargonium reniforme und Plantago major und/oder Plantago lanceolata unterstützend zu anderen Medikamenten genommen werden, die für die Indikation angezeigt sind. Diese unterstützende Einnahme kann in derselben Dosierungsform oder getrennt davon geschehen.

Im folgenden werden die bevorzugten einzelnen Bestandteile im Detail erläutet, wobei auf die Figuren Bezug genommen wird:

Dabei zeigt:
- **Figur 1**: Lineare Abhängigkeit der Adsorption von Gerbstoffen von der Konzentration. Differenz der optischen Extinktion einer Farbreaktion vor und nach Adsorption gegen die Konzentration von Chlorogensäure CHS und eines Gerbstoffprodukts CK (Gracza 1987, *infra*)
- **Figur 2**: Adsorptionsisotherme. Adsorbierte Menge a in Abhängigkeit von der Konzentration c strebt einem Sättigungswert zu (Näser 1974, *infra*)
- **Figur 3**: Vernetzung von Eiweißmolekülen durch Polyphenol-Gerbstoffe. a) Ausbildung von Monolayer bei niedriger Proteinkonzentration. b) Vernetzung der Proteine bei hoher Eiweißkonzentration
- **Figur 4**: Bindung von Pflanzenschleim an die Oberfläche der Mundschleimhaut. A) Mikroskopischer Schnitt durch die Mundschleimhaut. B) Fluoreszenzmikroskopische Aufnahme der Anlagerung von Rhamnogalacturonan ausschließlich an die Oberfläche der Mundschleimhaut (Schmidgall 2000, *infra*).
- **Figur 5**: Adsorptionskinetik von Pflanzenschleim (Rhamnogalacturonan) an die Mundschleimhaut, Konzentration an Rhamnogalacturonan in der Inkubationslösung [mg/ml] gegen die adsorpierte Menge [mg/cm² ] (Schmidgall 2000, *supra*); im Vergleich die theoretisch abgeleitete Langmuir'sche Kurve (Näser 1974, *infra*).

### Pelargonium-Extrakt

Beim dem bevorzugten Bestandteil Pelargonium-Extrakt, einem wässrig-ethanolischen Auszug aus der Wurzel von *Pelargonium sidoides* in Form einer Urtinktur steht für die Lutschpastille die oben erklärte Gerbstoff-Wirkung im Vordergrund.

### Systematik der Gerbstoffe

Unter Gerbstoffen verstand man ursprünglich Pflanzenauszüge, die zum Gerben tierischer Haut und zur Lederherstellung verwendet wurden. Da die Lederindustrie hierfür heute überwiegend chemische Substanzen verwendet, interessiert nur mehr die adstringierende Wirkung dieser Naturstoffklasse in Pharmazie und Kosmetik.

Diese Adstringenswirkung beruht auf einer Wechselwirkung mit Eiweißmolekülen der Kollagenfasern der obersten Gewebeschichten der Haut und Schleimhäute. Diese Wirkung zeigen nur Gerbstoffe mit einem Molekulargewicht zwischen 500 und 3000 und ein bis zwei Hydroxylgruppen pro 100 Masseneinheiten.

Aus ökologischer Sicht gesehen, bilden Pflanzen Gerbstoffe als sekundäre Inhaltsstoffe um Fressfeinde (Pflanzenfresser, Insekten) und phytopathogene Infektionserreger (Pilze, Bakterien, Viren) abzuwehren.

Aufgrund chemischer Eigenschaften unterteilt man die Stoffklasse in kondensierte und hydrolysierbare Gerbstoffe.

### Kondensierte Gerbstoffe:

Dazu zählen die Catechingerbstoffe und die Proanthocyanidine.

Bausteine der kondensierten Catechingerbstoffe sind die Flavanole (+)-Catechin und (+)-Epicatechin. Es treten auch Mischkondensate auf, die zusätzlich Leucoanthocyanidine enthalten und als Proanthocyanidine bezeichnet werden.

Besonders kondensierte Gerbstoffe neigen zu Oxidation und Polymerisation, was letztlich zu wasserunlöslichen, therapeutisch wertlosen Substanzen mit hohen Molekulargewichten führt. Diese sind auch analytisch nicht mehr erfassbar und geben sich durch braune Färbung (sog. "Gerbstoffrote") zu erkennen.

Für die weitere Betrachtung sind daher nur die Oligomeren dieser Substanzklasse wichtig.

### Hydrolysierbare Gerbstoffe:

Diese Gerbstoffe lassen sich durch Hydrolyse in Zucker und Gallussäure, bzw. Ellagsäure (Hexahydroxydiphensäure) spalten. Daher unterscheidet man Gallotannine und Ellagitannine.

Typischer Vertreter ist das "Tannin" (Hexagalloylglucose) oder die Digallussäure. Häufig treten auch Mischverbindungen auf, die bei Hydrolyse sowohl Gallussäure, als auch Catechin ergeben, wie z.B. die Epicatechingallate aus Schwarzem Tee.

### Gerbstoffe in Pelargonium sidoides:

Der Gesamt-Gerbstoffgehalt in den Wurzeln von *Pelargonium sidoides* beträgt ca. 9% in der Trockenmasse (TM) (Kolodziej, H., Kayser, O. Pelargonium sidoides DC - Neueste Erkenntnisse zum Verständnis des Phytotherapeutikums Umckaloabo. Z. Phytother. 19: 28 (1998)). Dabei ist der Gehalt an hydrolysierbaren Gerbstoffen sehr gering, da die gefundenen Werte für Gallussäure kleiner 0,01% in TM und für Gallussäure-methylester ca. 0,02 % in TM betragen (Kayser, O.: Phenolische Inhaltsstoffe von P. sidoides. Diss. FU Berlin D188, 1997).

Es dominieren demnach Catechingerbstoffe, für die als Monomere meist Catechin, und Gallocatechin, in geringem Umfang auch Afzelechin nachgewiesen wurden (Kolodziej 1998, supra). Diese Monomeren liegen in einem komplex zusammengesetzten Gemisch polymerer Strukturen von mindestens acht Flavonoleinheiten vor, deren Molekulargewicht ca. 2300 beträgt und damit noch im Bereich einer adstringierenden Wirkung liegt.

### Wirkmechanismus: Adsorption von Gerbstoffen an Proteine

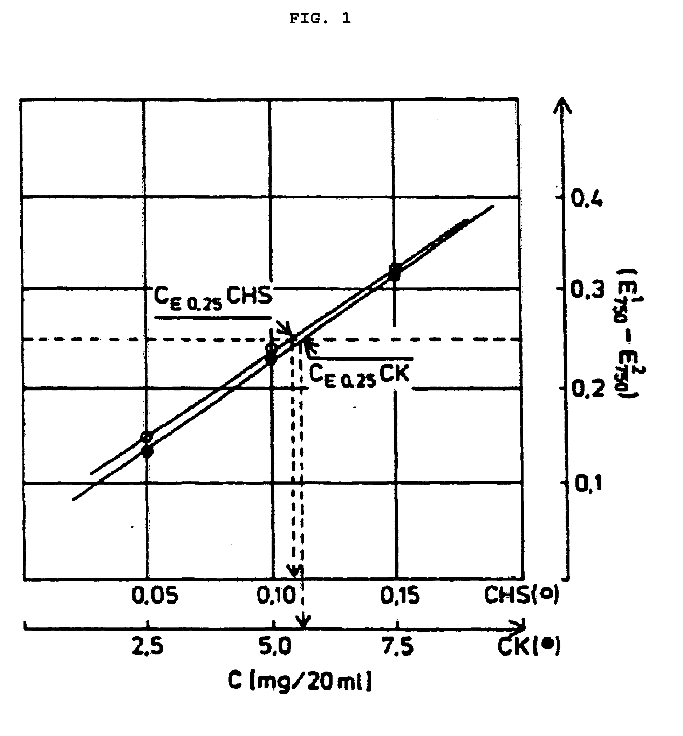

Beim Kontakt von Gerbstoffen mit Eiweißmolekülen kommt es zunächst zu einer Adsorption der Substanz an die offenen ("amorphen"), nicht quasikristallinen Abschnitte der Eiweißmoleküle. Für geringe Konzentrationen von Adstringens im Vergleich zur Menge an Eiweiß beobachtet man eine lineare Abhängigkeit der Adsorption von der Konzentration (Gracza L., Adstringierende Wirkung von Phytopharmaka. Dtsch. Apotheker Ztg. 127, 2256-2258, 1987, siehe Figur 1).

Dieser Sachverhalt einer linearen Beziehung zwischen Gerbstoffadsorption und Konzentration wird in der Analytik der Gerbstoffe genutzt. Durch einen hohen Überschuss an Hautpulver (Proteine) wird der Gerbstoff vollständig gebunden und durch die Differenz der optischen Extinktion einer Farbreaktion vor und nach der Adsorption 1E1(750) - E2(750)j dargestellt (Grazca, 1987, supra).

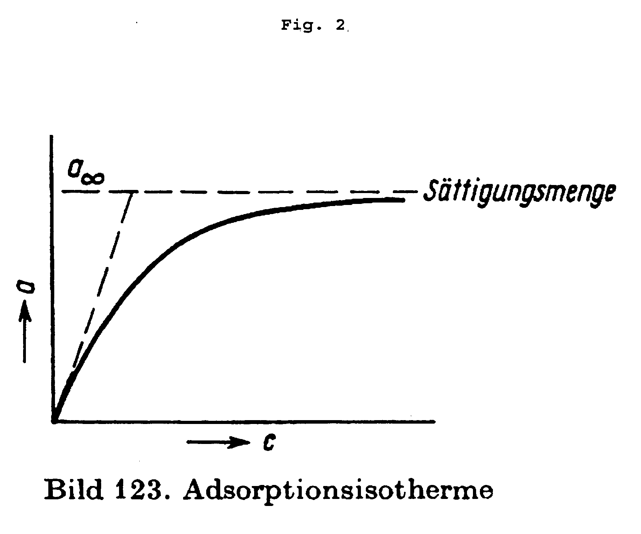

Bei weiterer Zugabe von Gerbstoff strebt der Adsorptionswert einem Grenzwert zu. Die hieraus resultierende Kurve (Adsorption gegen Konzentration aufgetragen) ist völlig verschieden von einer pharmakologischen Dosis-Wirkungs-Beziehung. Die vorliegenden Verhältnisse sind aus der physikalischen Chemie bekannt und werden üblicherweise durch die Langmuir'sche Gleichung für Adsorptionsisothermen beschrieben. Die oben in Fig. 1 wiedergegebene Grafik repräsentiert den unteren, linearen Teil der Kurve gemäß Figur 2.

Dann wird das Langmuir'sche Gesetz der Adsorption als Funktion der adsorbierten Menge a als Funktion der Konzentration c dargestellt.

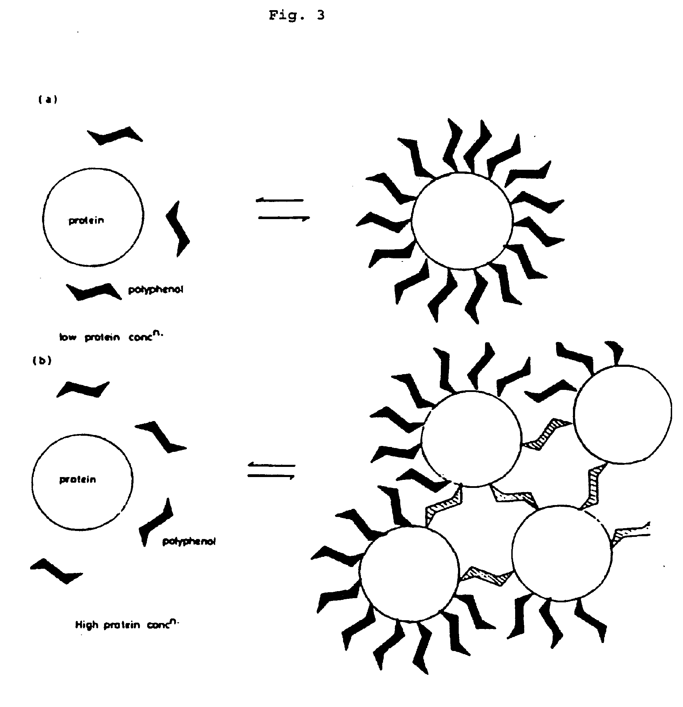

Der hierdurch beschriebene Sättigungswert resultiert aus der Ausbildung von "Monolayers" und damit einer Sättigung der verfügbaren Oberfläche der Eiweißmoleküle mit Gerbstoff (siehe Fig. 3).

Darin wird die Bildung von "monolayers" durch Gerbstoffe auf Eiweißmolekülen und Vernetzung von Proteinen durch diese Gerbstoffschicht beschrieben. (Haslam E, Plant polyphenols, Vegetable tannings revisited, Cambridge University Press, Cambridge 1966, Polyphenol complexation 154-219, S. 171)

Es handelt sich bei diesen Adsorptionsvorgängen um einen rein physikochemischen Vorgang, der für die Adstringens-Wirkung der Substanzklasse typisch ist.

Von Adstringens-Wirkung spricht man, wenn der Gerbstoff die Eiweißmoleküle (z.B. in Kollagenfasern) durch weniger stabile Ionen- oder Wasserstoffbrücken-Bindungen vernetzt.
Diese Vorgänge sind Grundlage der Wirkung der erfindungsgemäßen Lutschpastille auf die Schleimhäute im Mund, da wenig Gerbstoff und eine große Eiweißmenge vorliegt. Davon abzutrennen ist die Gerbung im eigentlichen Sinne (z.B. bei der Lederherstellung), wo ein Überschuss an Gerbstoff (meist Tannin) kovalente Bindungen mit den Säureamidgruppen der Proteine eingeht, was zur Aufnahme etwa der Hälfte des Ledergewichts an Tannin und damit zur Sättigung führt. Dieser Zustand kann aufgrund der sehr geringen Menge an Gallussäure in der Dosierung der Lutschpastille nicht erreicht werden.

Daher kann man davon ausgehen, dass die in der Lutschpastille vorhandene Menge an Gerbstoffen zwar an die Mundschleimhaut gebunden wird, aber nicht die Epithelbarriere durchdringen kann und damit auch nicht resorbiert wird.

Dadurch unterscheidet sich die hier erfindungsgemäß beanspruchte Verwendung der Zusammensetzungen auch deutlich von früher beschriebenen Zusammensetzungen, die Pelargonium bzw. Pelargonium-Extrakte enthalten, die sämtlich auf systemische Wirkungen abzielen und daher auch höhere Dosierungen benötigen.

### Zusammenfassung

Die aus Pelargonium-Extrakt der Lutschpastille freigesetzten Gerbstoffe vernetzen Proteine der entzündeten Schleimhaut in Mund und Rachen aufgrund ihrer adstringierenden Eigenschaften. Dabei handelt es sich um rein physikochemische Wirkmechanismen der Adsorption und Ionenbindungen, die definitionsgemäß nicht zu pharmakologischen Wirkungen gezählt werden. Aufgrund des Wirkmechanismus der oberflächlichen Adsorption wird Gerbstoff im Mundraum auch nicht resorbiert.

Eine weitere Wirkung entfalten Gerbstoffe z. B. wenn sie im Darm freigesetzt werden, da sie eine stark antibakterielle Wirkung aufweisen.

### Spitzwegerich-Extrakt

Beim Bestandteil Spitzwegerich-Extrakt steht für das erfindungsgemäße Präparat die einhüllend-abdeckende Wirkung der Schleimstoffe im Vordergrund.

### Schleimstoffe

Richtigerweise sollte man von Pflanzenschleimen sprechen, die durch Extraktion mit heißem oder kaltem Wasser aus Pflanzenmaterialien gewonnen werden. Diese Schleimpolysaccharide sind in der Regel Heteropolysaccharide, also polymere Kohlenhydrate aus glycosidisch verknüpften Zuckern, die selbst in hochviskosen Lösungen nicht kleben. Je nach Anteil von Uronsäurebausteinen können diese Hydrogele neutral oder sauer reagieren. Aufgrund der Primärstuktur lassen sich die Polymere einteilen in Mannane, Glucomannane, Galactomannane, Xylane, Rhamnogalacturone usw., wobei in schleimbildenden Pflanzen meist verschiedene chemisch unterscheidbare Biopolymere vorkommen. Das Molekulargewicht der Schleimpolysaccharide liegt in der Größenordnung 5 x 10⁴ - 10⁶.

### Schleimstoffe in Spitzwegerich

In den Blättern von Spitzwegerich *Plantago lanceolata* finden sich 2 - 5% an Schleimpolysacchariden, bestehend aus den Monomeren Glucuronsäure 7%, Galacturonsäure 31%, Galactose 44%, Arabinose 32%, Glucose 9%, Rhamnose 7%, Mannose 4%, Fucose und Xylose.

Der Rohschleim lässt sich weiter in vier Fraktionen auftrennen, die aus einer neutralen und mehreren sauren Polysaccharidfraktionen bestehen.

### Neutrale Polysaccharide

Arabinogalactan und Glucomannan Struktur:

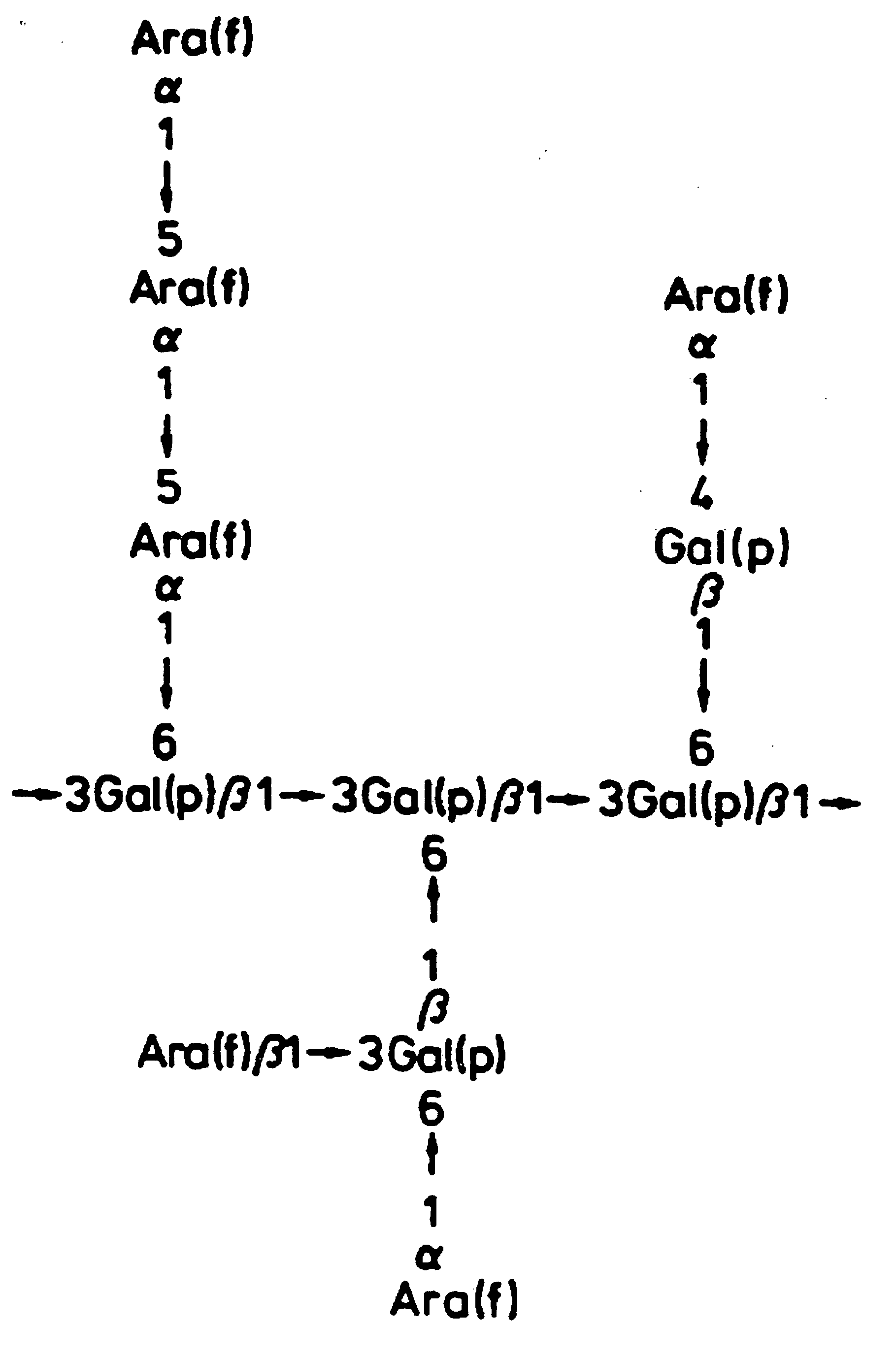

### Saure Polysaccharide

Rhamnogalacturonan mit Arabinogalactan-Seitenketten von folgender Struktur: 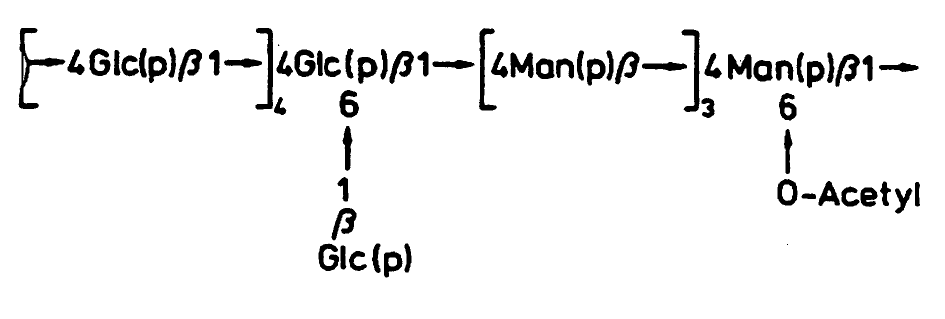 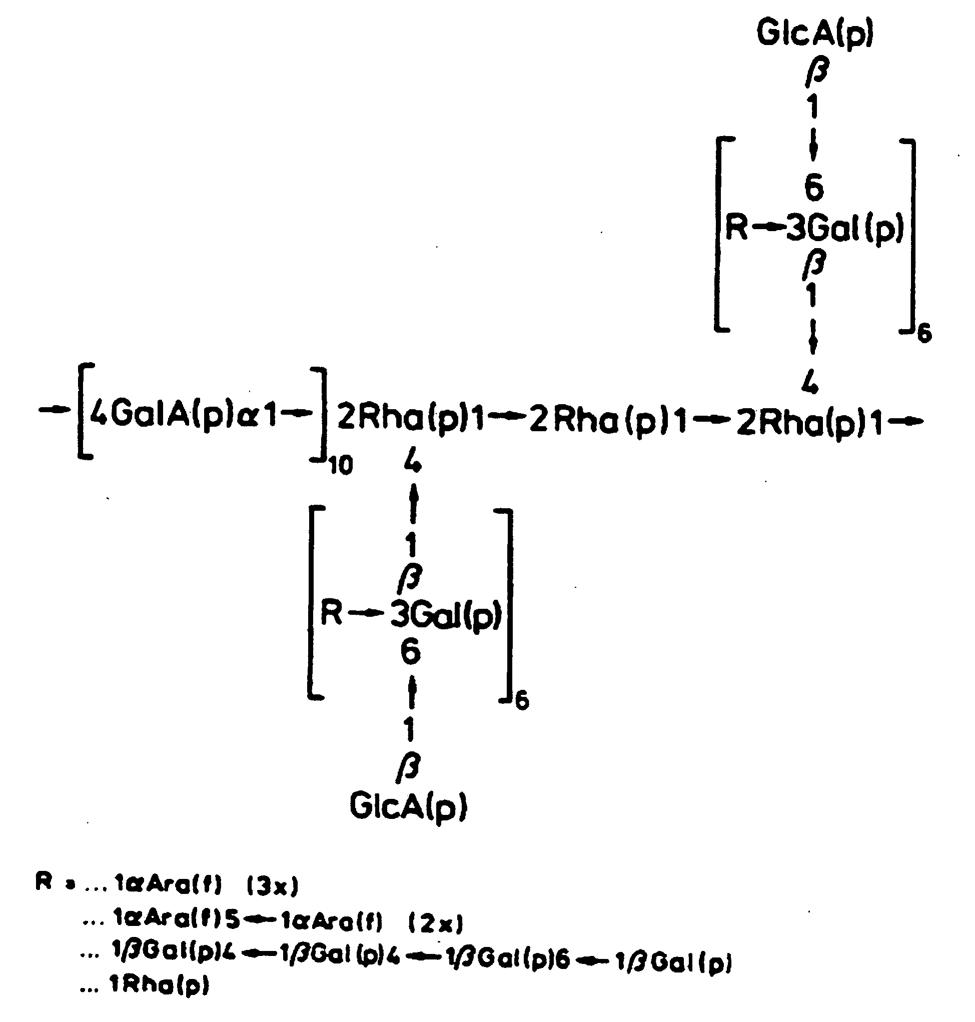 sowie drei weiteren sauren Fraktionen mit Molekulargewichten unter 10.000.

Daneben enthält Spitzwegerich 2-4 % Iridoide mit den Hauptkomponenten Aucubin und Catalpol, sowie Gerbstoffe, Flavonoide, Phenolcarbonsäuren (Kaffeesäure, Ferulasäure) und Phenylethanoide, wie Acetosid (Paper DH, Marchesan M, Spitzwegerich (Plantago lanceolata L.) Z. Phytother. 20, 231-238, 1999).

### Wirkmechanismus: Adsorption von Schleimstoffen an die Mundschleimhaut

### Aufbau und Funktion der Mundschleimhaut

Die Anatomie der Mundschleimhaut zeigt eine Epithelschicht, die von einer Bindegewebsschicht (*Lamina propria*) gestützt wird, von der sie durch eine Basalmembran getrennt ist. Je nach Lage im Mundraum werden verschiedene Typen von Schleimhautepithel unterschieden. Am Zahnfleisch und Gaumen ist diese keratinisiert. Die übrige Schleimhaut - außer am Zungengrund- zeigt 4 Schichten (superficial, intermediate, prickle-cell und basal layer). Die Dicke des Epithels beträgt 500 - 600 pm, bei einer Gesamtfläche von ca. 50 cm². Die "membrane-coating granules" der "prickle-cells" sind lipidhaltige Organellen, deren Inhalt in die Interzellularräume entlassen wird und dort für den Zellzusammenhalt und die Interzellularbarriere zuständig ist. Die Mundschleimhaut besitzt keine dichtschließenden Haftkomplexe ("tight junctions") zwischen den Epithelzellen, wie sie für die intestinale Schleimhaut (im Darm) typisch sind.

Die Schleimschicht der Mundschleimhaut befeuchtet und schützt das Epithel und macht es gleitfähig. Der eigentliche Schleim, Mucin genannt, ist ein Glycoprotein, dessen Eiweißkern von Oligosaccharid-Seitenketten umgeben ist. Dadurch wird auch der pH-Wert von 5,8 - 7,4 des Speichels bedingt.

Von den Speicheldrüsen werden pro Tag 1000 - 1500 ml Speichel sezerniert (ca. 40-60 ml/Std) (Roche Lexikon Medizin, © Urban & Fischer, 5. Aufl. 2003, im Internet unter http://www.gesundheit.de/roche/ aufgerufen am 5.6.2005).

Damit stehen in den 10 Minuten, in denen etwa eine Pastille gelutscht wird, bis zu 10 ml Speichel zur Verfügung.

Auch im Speichel sind physiologische Schleimsubstanzen vorhanden. Neben Glycoproteinen vom Mucintyp sind 3 Typen von Polysacchariden in einer Konzentration von 50-300 mg/100ml enthalten. Fucomucoide enthalten Fucose, Hexosamin, Galactose und etwas Mannose, während die Sialomucoide reich an Sialinsäure und Acetylgalactosamin sind. Die in geringer Menge vorkommenden sauren Glycosaminglycane sind noch nicht genauer untersucht.

Bei Infektionen und Entzündungen im Mundraum nimmt allgemein der Speichelfluß stark ab.

### Wirkmechanismus von Schleimstoffen

Für verschiedene Pflanzenschleime wurde an in-vitro-Modellen deren Bindung an die Mundschleimhaut untersucht.

Die Bindung des Schleims an die kultivierten Mundschleimhautzellen kann durch Abnahme der Konzentration an Polysacchariden in der überstehenden Lösung gemessen werden. Das Ausmaß und die Geschwindigkeit des Effekts ist abhängig von der verwendeten Schleimdroge.

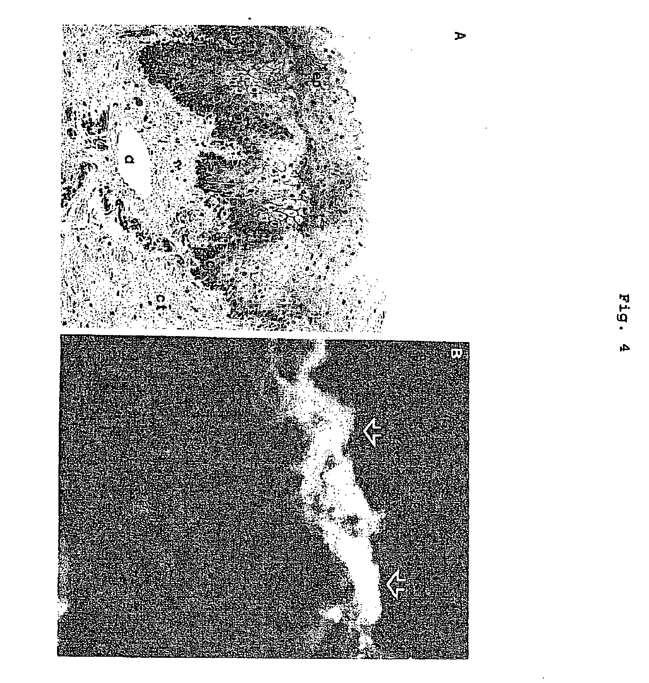

In Fig. 4 ist die Bindung des Pflanzenschleims an Mundschleimhautzellen gezeigt (histochemische Untersuchung). Das dabei verwendete fluoreszenz-markierte Rhamnogalacturonan wird nur apikal an das Epithel angelagert [ep: Epithel, ct: Bindegewebe, bc: Basalzellen, d: Lymphgefäß] (Schmidgall J. Schnetz E, Hensel A., Evidence for bioadhesive effects of polysaccharides and polysaccharide-containing herbs in an ex vivo bioadhesion assay on buccal membranes, Planta Med. 2000 Feb; 66(1):48-53)

Es zeigt sich an diesen Untersuchungen, dass der Pflanzenschleim sich nur an die apikalen Teile des Mundschleimhautepithels anlagert.

Darüberhinaus wurde auch *in vivo* eine nur oberflächliche Bindung von Pflanzenschleimen an Schleimhaut am Modell der Magenschleimhaut der Ratte mit vergleichbaren Hydrocolloiden aus Opuntia belegt (Galati EM, Pergolizzi S, Miceli N, Monforte MT, Tripodo MM., Study on the increment of the production of gastric mucus in rats treated with Opuntia ficus indica (L.) Mill. Cladodes, J. Ethnopharmacol. 2002 Dec;83(3):229-33).

Der Mechanismus, der dieser Bindung von Pflanzenschleim an Epithelien zugrunde liegt, wird in dieser Untersuchung ausdrücklich als Adsorption bezeichnet (Schmidgall 2000 supra).

Die Bindung des Schleims an die kultivierten Mundschleimhautzellen kann durch Abnahme der Konzentration an Polysacchariden in der überstehenden Lösung gemessen werden. Die Adsorption der Pflanzenschleime an die Zellen ist demnach ein konzentrationsabhängiger Prozess, dessen Kinetik dem Bild einer Adsorptionsisotherme entspricht (Schmidgall 2000 supra).

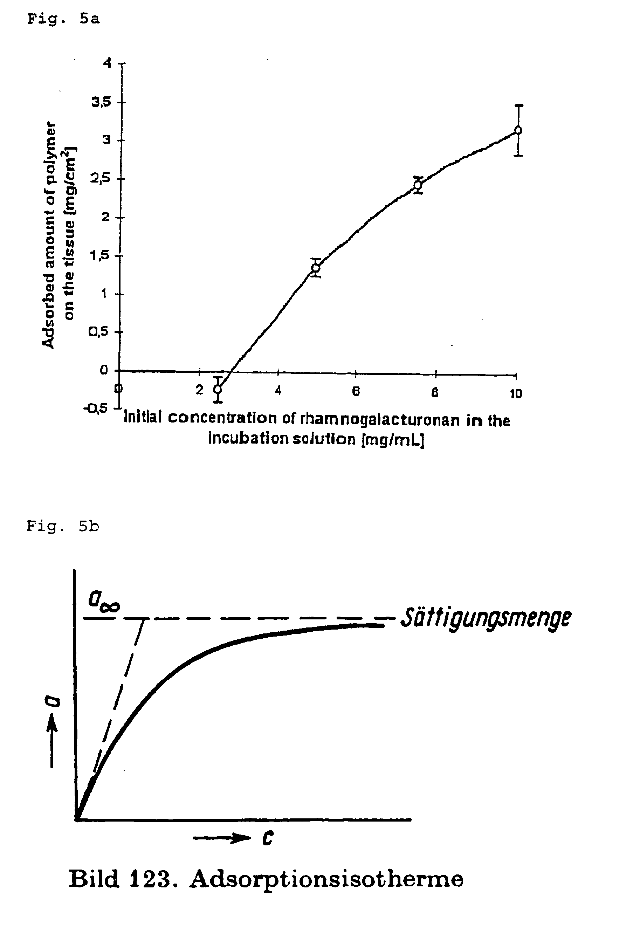

In Fig. 5 wird die
a) Adsorptionskinetik von Planzenschleim (Rhamnogalacturonan) an die Mundschleimhaut (Schmidgall 2000 supra) und
b) zum Vergleich die theoretische abgeleitete Langmuir'sche Kurve [c=Konzentration, a=adsorbierte Menge] (Näser KH: Physikalische Chemie, VEB Verlag für Grundstoffindustrie, Leipzig 1974) gezeigt.

Die hieraus resultierende Kurve (Adsorption gegen Konzentration aufgetragen) ist völlig verschieden von einer pharmakologischen Dosis-Wirkungs-Beziehung. Die vorliegenden Verhältnisse sind aus der physikalischen Chemie bekannt und werden üblicherweise durch die Langmuir'sche Gleichung für Adsorptionsisothermen wiedergegeben.

Es handelt sich bei diesen Adsorptionsvorgängen um einen rein physikochemischen Vorgang, der für die mucoprotektive Wirkung der Substanzklasse Schleimpolysaccharide typisch ist. Wie die Abbildung der *in vitro*-Versuche zeigt, lagert sich der Pflanzenschleim nur an die Oberfläche des Epithels an. Der Schleim durchdringt aber nicht die Mundschleimhaut und wird daher nicht resorbiert. Nach einiger Zeit löst sich diese Schleimschicht auch wieder ab.

### Zusammenfassung

Die aus Spitzwegerich-Extrakt des Medizinprodukts freigesetzten Schleimstoffe legen sich aufgrund ihrer bioadhäsiven Eigenschaften als Film auf die entzündete Schleimhaut in Mund und Rachen. Dabei handelt es sich um rein physiko-chemische Wirkmechanismen der Adsorption, die definitionsgemäß nicht zu pharmakologischen Wirkungen gezählt werden. Aufgrund des Wirkmechanismus der reversiblen Adsorption an die Epitheloberfläche werden Schleimstoffe im Mundraum auch nicht resorbiert.

Als mögliche Anwendungsgebiete der gegenwärtigen Erfindung sind daher Mund- und Rachentherapeutika allgemein zu nennen.

Eine solche Verwendung wäre dann angezeigt, wenn die Schleimhäute des Mund-Rachen-Raums durch die obligate, natürliche Keimflora der Mundhöhle (10E7 bis 10E9 Bakterien pro Milliliter Speichel) aufgrund einer viralen Infektion oder mechanischer bzw. chemischer Reize infiziert werden.

Durch diesen Vorgang kommt es zu entzündungsbedingten Schmerzzuständen, insbesondere beim Schlucken. Die hierfür nötige symptomatische Behandlung besteht aus einer Linderung dieser Zustände mit dem Ziel, das subjektive Befinden des Patienten zu bessern.

Präparate zum Lutschen oder zum Gurgeln sind für das Anwendungsgebiet gebräuchlich.

Weitere Anwendungsgebiete sind weitere Entzündungen, die durch eine topische Behandlung im obigen Sinne gelindert werden können, z.B. Entzündungen der Speiseröhre, des Magens und der Haut. Dabei wird der erfindungsgmäße Extrakt bzw. die erfindungsgemäße Kombination vorzugsweise in die üblicherweise verwendete Darreichungsform formuliert, jedoch insbesondere eine solche, die eine topische Darreichung der Erfindung an die entzündete Stelle ermöglicht.

Die gegenwärtige Erfindung enthält die Gerbstoff-haltige Pflanze Pelargonium sidoides und/oder Pelargonium reniforme und die Schleim-haltige Pflanze Plantago major und/oder Plantago lanceolata.

Dabei sollen die Gerbstoffe Proteine der entzündeten Schleimhaut in Mund und Rachen vernetzen. Infektionen wird so vorgebeugt. Die Pflanzenschleime umhüllen die Schleimhäute im Mund mit einem schützenden Film.

### Zusammenfassend kann festgehalten werden:

Adstringentia führen zu einer Verdichtung des kolloidalen Gefüges und zu einer oberflächlichen Abdichtung auf der Schleimhaut des Mund-Rachenraumes. Da das Konzentrationsverhältnis von Gerbstoffmolekülen zu Eiweiß sehr klein ist, kommt es nur zu einer oberflächlichen Komplexierung der Eiweißmoleküle über die Polyphenolmoleküle und damit zur Bildung einer zusammenhängenden und schützenden Membran. Damit wird die Reizung der Schleimhaut vermindert und ein milder antibakterieller und anästhesierender Effekt erzielt.

Durch entzündliche Prozesse an der Schleimhaut im Mund-Rachenraum wird der endogene Schleimbelag des Epithels in seiner Funktion gestört, was zu chemischer und mikrobieller Reizung führt. Der Effekt von Pflanzenschleimen bei dieser Anwendung ist, dass sich die Schleimpolysaccharide dünn an den oberen Schichten der Mundschleimhaut anlagern und so auf einer entzündeten Mundschleimhaut einen - der natürlichen Schleimschicht physiologisch ähnlichen - Schutzfilm bilden. Dadurch kommt es zu Rehydration, Desensibilisierung der Schmerzrezeptoren und Abnahme der lokalen Entzündung mit der Folge einer verminderten Hustenreizung.

Diese beiden Effekte werden z.B. durch die Anwendungsform einer Pastille bevorzugt in synergistischer Weise optimal erfüllt, da durch das langsame Lutschen die Wirkstoffe in intensiven Kontakt mit der Mundschleimhaut treten. Die Wirkstoffe gehen sofort in den Lösungszustand über, da z.B. Arabisches Gummi - Pastillen feste Lösungen darstellen.

Der Grundstoff Arabisches Gummi (Akaziengummi, Acacia senegal), aus dem die Pastillen bevorzugt gefertigt werden, bildet mit Wasser bis zu 50%ige kolloidale Lösungen, die keine Neigung zur Gelbildung zeigen, sondern kleben. Aus den hochverzweigten Arabinogalactanen des Arabisches Gummi bilden sich mit Wasser hochviskose hydrocolloide Lösungen, die im Mund das Schleimgefühl von Spitzwegerich noch verstärken.

Zusätzlich wird durch eine Pastille ein mechanischer Reiz ausgelöst, der zusammen mit der sauren Citronensäure und den Hilfsstoffen Maltitol und Sorbitol einen sog. sialagogen Effekt hervorruft, d.h. die Speicheldrüsen zur Sekretion angeregt. Dabei wird zusätzlich die Mundschleimhaut gespült und natürlicher Schleim aus dem Speichel im Mund verteilt.

### Bevorzugte Zusammensetzung einer Lutschpastille

Die Erfinder haben bei der Entwicklung der Darreichungsform festgestellt, dass die Urtinktur direkt in Pastillen eingearbeitet werden kann; dann wird die den 2mg DE äquivalente Menge von 100 mg Urtinktur eingesetzt. Die Urktinktur enhält dabei lediglich 1% der Trockensubstanz. Dies sollte bei der Interpretation der eingesetzten Mengen an Urtinktur von Pelargonium und Spitzwegerich mit in die Betrachtung einbezogen werden.

Bei einer Tagesdosis der Lutschpastille von 6 Pastillen ergibt dies 600 mg Urtinktur/d, was 40% einer Urtinkturdosierung (homöopathisches Arzneimittel) entspricht.

Damit entspricht die Tagesdosis der Lutschpastille (6 Pastillen) 16 bis 25% der arzneilich wirksamen Dosis des Phytopharmakons.

Für das erfindungsgemäße Produkt wirksamkeitsbestimmend ist der Gerbstoffgehalt und Schleimstoffgehalt der Pastillen.

Getrocknete *Pelargonium sidoides -* Wurzeln enthalten ca. 9% Gerbstoffe (Kolodziej 1998, supra).

Bei einem DEV von 6,3 : 1 für den DE ergibt sich:
9 g Gerbstoff in 100 g Droge entspr. 15,87 g DE.

Damit sind in 2 mg DE/Pastille, bzw. 100 mg Urtinktur 1,13 mg Gerbstoff enthalten.

Diese Menge pro 10 ml gelöst zeigt experimentell durchaus gute Wirkung als Adstringens. Deshalb kann von einer angemessenen Dosierung ausgegangen werden, da in den 10 Minuten, in denen etwa eine Pastille gelutscht wird, durchschnittlich bis zu 10 ml Speichel zur Verfügung stehen.

### Pharmakologische Bewertung von Spitzwegerich-Extrakt

Dieser Bestandteil repräsentiert die Schleimstoffwirkung des erfinderischen Produktes.

Laut Kommission E - Monographie zeigt Spitzwegerich folgende Wirkungen: reizmildernd, adstringierend, antibakteriell. Als Tagesdosis werden 3 - 6 Droge angegeben.

Ein handelsüblicher Trockenextrakt hat ein DEV von 4-5 : 1.

Damit entsprechen 3 g Droge = 0,6 - 0,75 g TE/d 6 g Droge = 1,2 - 1,5 g TE/d, in Summe 0,6 - 1,5 g TE/d.

Mit der Vorgabe von 6 Pastillen/Tag und der Dosierung von 10% der Angabe in der Kommission E-Monographie ergibt sich:
0,06 - 0,15 g TE/d = 60 - 150 mg TE/d oder 10 - 25 mg TE/Pastille.

### Interaktionen

Zu therapeutischen Interaktionen der Bestandteile Pelargonium *sidoides* und Spitzwegerich sind im Stand der Technik keinerlei Hinweise vorhanden. Tatsächlich existiert im Stand der Technik ein Vorurteil gegen eine Kombination von Pelargonium und Spitzwegerich, da Pelargonium Gerbstoffe enthält und Spitzwegerich Schleimstoffe, von denen bekannt ist, dass sie ausfallen, wenn sie zusammen verwendet werden. Erst mit der vorliegenden Erfindung werden die besonders bevorzugten Eigenschaften dieser Kombination deutlich.

Unverträglichkeit der physikalisch-chemischen Wirkstoffe des Medizinproduktes mit anderen Bestandteilen der Darreichungsform (Hilfsstoffe) sind nicht bekannt und aufgrund des Inhaltsstoffmusters der Pflanzen auch nicht zu erwarten.

### Beispiele

### Beispiel 1

1 erfindungsgemäße Lutsch-Pastille enthält beispielsweise:

| **Nr.** | **Bestandteil** | **Menge pro Pastille** | **Qualitäts-monographie** | **Funktion** |
|---|---|---|---|---|
| 1 | Wirkstoff | | | |
| 1 | Pelargonium Urtinktur | 100,00 mg | Lichtwer Spezifikation | Wirkstoff |
| 2 | Plantago lanceolata Trockenextrakt | 25,00 mg | Lichtwer Spezifikation | Wirkstoff |
| | Sonst. Bestandteile | | P | |
| 3 | Arabisches Gummi | 411,76 mg | Ph. Eur. curr. ed. | Strukturgeber |
| 4 | Maltitol-Lösung | 553,06 mg | Ph. Eur. curr. ed. | Zuckeraustauschstoff |
| 5 | Sorbitol Lösung 70% (nicht-krist.) | 142,85 mg | Ph. Eur. curr. ed. | Zuckeraustauschstoff |
| 6 | wasserfreie Citronensäure | 6,50 mg | Ph. Eur. curr. ed. | Säuerungsmittel |
| 7 | Menthol | 0,80 mg | Ph. Eur. curr. ed. | Geschmackskorrigens |
| 8 | Acesulfam-Kalium | 0,5 mg | Ph. Eur. curr. ed. | Süßungsmittel |
| 9 | Geraniumöl, natürlich | 0,05 mg | Frey+Lau | Geschmackskorrigens |
| 10 | Dünnflüssiges Paraffin | 1.30 mg | Ph. Eur. curr. ed. | Glänzmittel |
| 11 | Gebleichtes Wachse | 0.07 mg | Ph. Eur. curr. ed. | Glänzmittel |
| 12 | Prozess-Wasser | q.s. | Bolder Spezifikation | Lösungsmittel |
| | Pastillengewicht | 1000.00 mg | | |

### Beispiel 2

### Herstellungsverfahren der Extrakte

### (A) Pelargonium

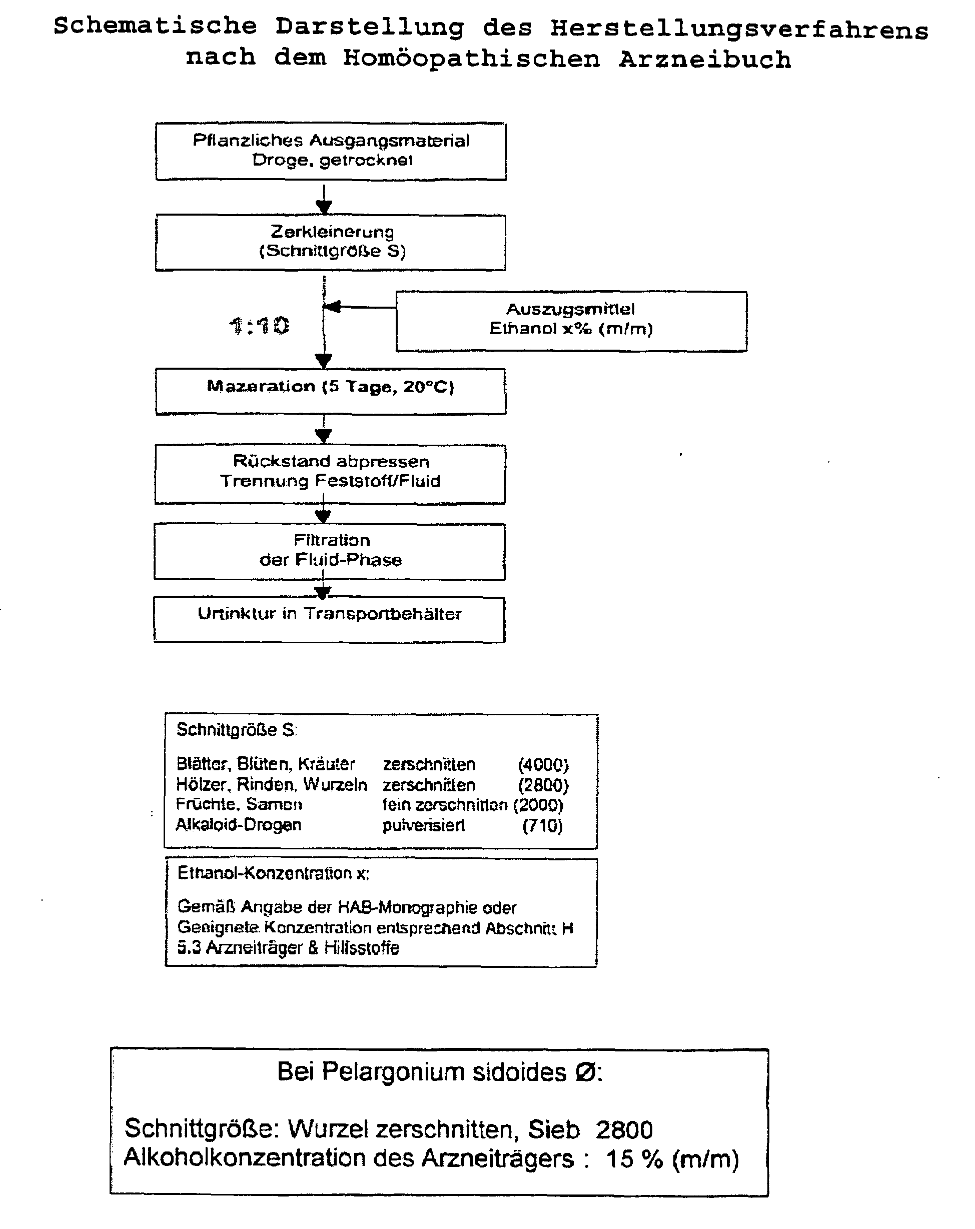

### (B) Spitzwegerich

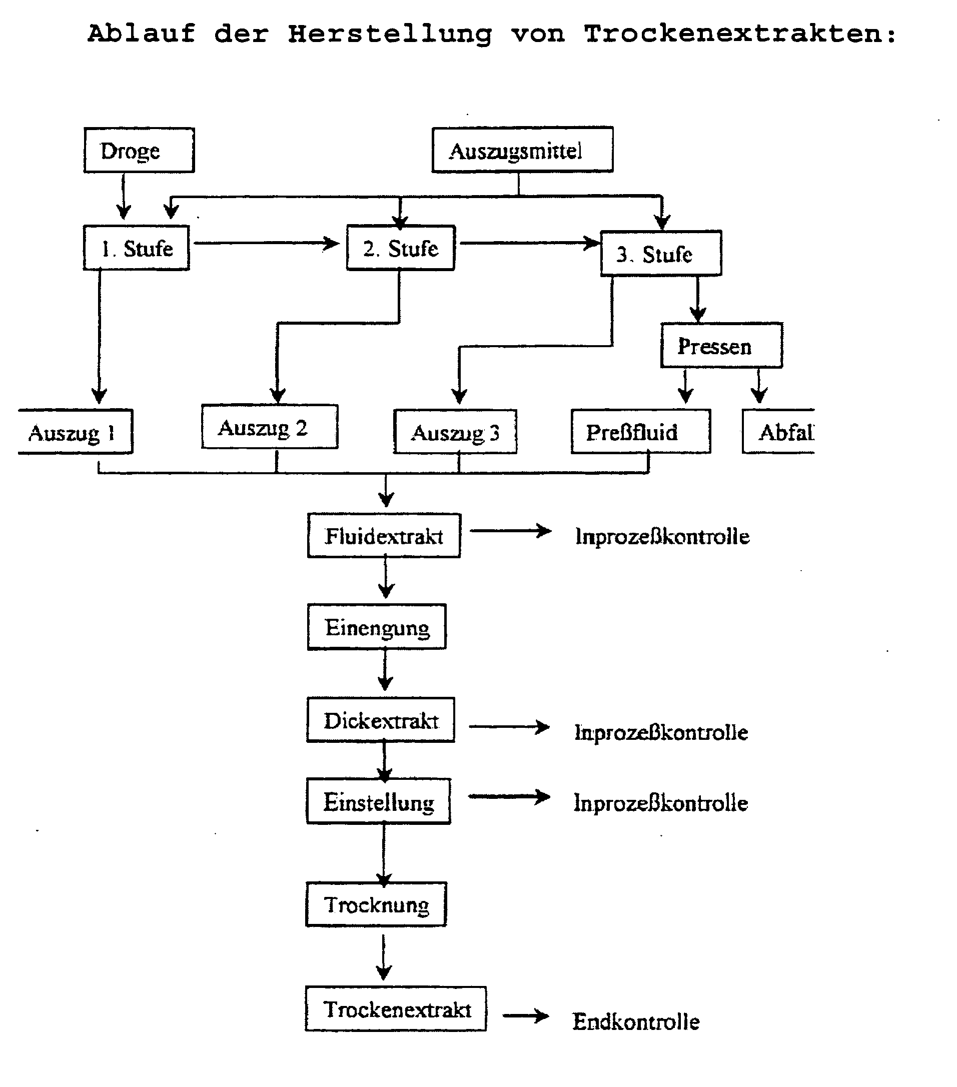

Die Herstellung der Pastillen basiert hier auf Verwendung Arabischen Gummis oder anderen Hydrokolloiden. In diesen Grundansatz werden Wirk- und Hilfsstoffe auf bekannte Weise eingearbeitet. Die entstehende sirupartige Masse wird auf einer so genannten Mogul-Anlage in vorgefertigte Puderformen gegossen und anschließend schonend getrocknet, ausgepudert und geglänzt.

### Beispiel 3

### Herstellung einer zweischichtigen Lutschpastille

Im folgenden wird die Produktion einer zweischichtigen Lutschpastille dargestellt, bei der es sich um eine besonders bevorzugte Ausführungsform der vorliegenden Erfindung handelt, die eine Freisetzung von zunächst Pelargonium und darauffolgend Spitzwegerich ermöglicht.

Um eine unterschiedliche Freisetzungsgeschwindigkeit zu erreichen, wird für die eine Schicht ein leichtlöslicher Zuckeralkohol und für die zweite Schicht ein schwerer löslicher verwendet.

Zusätzlich kann in der schwerlöslichen Schicht noch ein Viskositätserhöher eingesetzt werden. Hierfür kommen Cellulosederivate oder Arabisches Gummi in Betracht. Für die Tablettierung werden diese zur Granulierung der Extrakte eingesetzt, um die Fließfähigkeit der Tablettenmasse auf der Presse zu verbessern.

Die Technik der 2-Schicht-Tablette ist bei pharmazeutischen Lohnherstellern für Tabletten vorhanden.

Heute sind eine ganze Reihe von Zuckeralkoholen im Handel erhältlich. Diese unterscheiden sich in ihrer Löslichkeit beträchtlich, wobei das Spektrum bei 35°C (Mundtemperatur) von 25 g/100 ml bis 85 g/100 ml Wasser reicht.

### Rezeptur-Beispiele

### Wirkstoffe:

*Pelargonium sidoides* Urtinktur 100 mg, entspr. 1,0 bis 1,5 mg Trockenextrakt nativ Spitzwegerich-Trockenextrakt 25 mg (5:1, wässrig)

### Geschmackstoffe:

Wasserfreie Citronensäure
Menthol
Geranium (Geranienöl, natürlich)

### Hilfstoffe:

Äußere Schicht
Sorbitol, Xylitol, Saccharose, Maltitol
Xylitol schmeckt süß-kühlend und Maltitol angenehm süß.

Hierbei kann die Urtinktur auf Saccharose aufgezogen und getrocknet werden. Die Geschmacksstoffe werden in diese Schicht eingearbeitet.

Innere Schicht
Mannitol, Isomalt, (mit Einschränkung Erythritol, dieses kühlend).

Die beiden erstgenannten Stoffe schmecken überwiegend süß und harmonieren mit dem krautigen Geschmack von Spitzwegerichextrakt.

Zur technologisch nötigen Granulierung des Trockenextraktes wird Arabisches Gummi oder ein Cellulosederivat (z.B.

Hydroxypropylmethylcellulose, HPMC) verwendet, die zusätzlich die Viskosität erhöhen und das Schleimgefühl im Mund verstärken.

### Produktprofil

Zunächst schmeckt das Produkt süß-kühlend durch die Kombination von Xylit und Menthol mit einer exotischen Note durch das Geranium. Damit wird von der Gerbstoff-Wirkung von Pelargonium geschmacklich abgelenkt. Die Gerbstoffe vernetzen sehr rasch die Proteine der gereizten Schleimhaut und dichten sie damit ab. Die äußere Schicht der Pastille löst sich relativ rasch auf.

Anschließend wird die überwiegend süß-kräuterig schmeckende zweite Schicht mit den Schleimstoffen langsam aufgelöst. Die Pflanzenschleime, zusammen mit dem klebfähigen Arabisches Gummi legen sich wie ein Film auf die gereizten Schleimhäute des Mund-Rachen-Raumes und beruhigen so den gereizten Hals.

### Beispiel 4

### Pelargonium-Spitzwegerich-Lutschpastillen

Klinische Pilotstudie (12/2005)
Teilnehmer:
n=7 (5 Männer / 2 Frauen)
Alter:
18 - 60 (Durchschnitt: 43,4 Jahre)
Einschlusskriterium/Diagnose:
Katarrhalische Rachenentzündung im Rahmen einer Erkältungskrankheit oder Bronchitis

In einer offenen Pilotstudie erhielten sieben Teilnehmer pro Behandlungstag 6 (Pelargonium-Spitzwegerich-)Lutschpastillen über einen Zeitraum von insgesamt 5 Tagen (Tag 0 Einschluss und Behandlungsbeginn, Tage 2 und 4 Untersuchungstage). Die Tagesdosis beträgt bis zu 6 Lutschpastillen, die über den Tag verteilt je nach Bedarf langsam gelutscht werden sollen.

| Symptomatik | Tag 0 | Tag 2 | Tag 4 |
|---|---|---|---|
| Halskratzen / Schleimhautirritation | 2,7 | 0,7 | 0,1 |
| Ausgetrockneter Schlund | 2,7 | 0,6 | 0,4 |
| Räusperzwang | 1,1 | 0,3 | 0,0 |
| Fremdkörpergefühl | 1,7 | 0,6 | 0,0 |
| Schmerzhaftes Leerschlucken | 2,9 | 0,6 | 0,1 |
| Hustenreiz | 2,6 | 1,0 | 0,4 |
| Pharyngealer Husten | 1,6 | 0,4 | 0,4 |
| Summenscore SYMPTOME | 2,2 | 0,6 | 0,2 |

| Untersuchungsbefund | Tag 0 | Tag 2 | Tag 4 |
|---|---|---|---|
| Diffuse Schleimhautverdickung | 1,7 | 0,9 | 0,1 |
| Schleimhautrötung, insbes. am hinteren Gaumenbogen | 2,3 | 0,7 | 0,4 |
| Geschwollene, dunkelrote Uvula | 1,6 | 0,7 | 0,0 |
| Fieber >38° C | 1,3 | 0,3 | 0,0 |
| Geschwollene, schmerzhafte Lymphknoten am Hals | 1,4 | 0,3 | 0,0 |
| Summenscore BEFUNDE | 1,7 | 0,6 | 0,1 |

### Beschwerdenscore:

0 =beschwerdefrei (bzw. kein Fieber)
1 = leichte Symptome
2 = mittelschwere Symptome
3 = schwere Symptome

Es wird deutlich, dass sich alle getesteten Beschwerden unter Einnahme der Pelargonium-Spitzwegerich-Lutschpastillen deutlich verbesserten.

## Patentansprüche

1. Verwendung von Pelargonium in Kombination mit Plantago zur Herstellung einer pharmazeutischen Zusammensetzung in Form einer Lutschpastille oder Lutschtablette für die topische Prophylaxe und Behandlung von Entzündungen des Mund- und Rachenraums, wobei Pelargonium *Pelargonium sidoides* und/oder *Pelargonium reniforme* ist und Plantago *Plantago major* und/oder *Plantago lanceolata* ist, wobei Pelargonium in der Form eines Extraktes verwendet wird.

2. Verwendung gemäß Anspruch 1, **dadurch gekennzeichnet, dass** zunächst die Wirkung des Pelargonium-Bestandteils und darauf folgend die Wirkung des Plantago-Bestandteils überwiegt.

3. Verwendung gemäß Anspruch 2, **dadurch gekennzeichnet, dass** eine zeitlich versetzte Wirkung der Bestandteile durch die verzögerte Freisetzung von Plantago bewirkt wird.

4. Verwendung gemäß einem der Ansprüche 2 oder 3, **dadurch gekennzeichnet, dass** die verzögerte Freisetzung durch Vermischen mit unterschiedlich schnell löslichen Substanzen bewirkt wird.

5. Verwendung gemäß einem der vorherigen Ansprüche, **dadurch gekennzeichnet, dass** auch Plantago in der Form eines Extraktes verwendet wird.

6. Verwendung gemäß Anspruch 5, **dadurch gekennzeichnet, dass** es sich bei dem Plantago-Extrakt um einen Trockenextrakt handelt.

7. Verwendung gemäß einem der vorherigen Ansprüche, **dadurch gekennzeichnet, dass** es sich bei dem Pelargonium-Extrakt um einen wässrig-ethanolischen Extrakt handelt.

8. Verwendung gemäß einem der vorstehenden Ansprüche, wobei es sich um eine zweischichtige Lutschpastille handelt, wobei eine innere Kernschicht durch den Plantago-Bestandteil gebildet wird und eine äußere Beschichtung durch den Pelargonium-Bestandteil gebildet wird.

9. Verwendung gemäß einem der vorstehenden Ansprüche, **dadurch gekennzeichnet, dass** es sich um eine Lutschpastille handelt und dass diese als weitere Bestandteile arabisches Gummi, Maltitol, Sorbitol, Xylitol, Saccharose, Isomalt, wasserfreie Citronensäure, Acesulfam Kalium, dünnflüssiges Paraffin, gebleichtes Wachs, Wasser, Cellulose-Derivate, Zuckeralkohole, Eudragit, und/oder Aromastoffe beinhaltet.

10. Verwendung gemäß Anspruch 9, **dadurch gekennzeichnet, dass** es sich bei den Aromastoffen um Geraniumöl und/oder Menthol handelt.

11. Verwendung gemäß einem der vorstehenden Ansprüche, wobei es sich um eine Lutschpastille handelt, die 80 - 200 mg Pelargonium-Urtinktur und 15 - 40 mg Plantago-Trockenextrakt umfasst.

12. Verwendung gemäß Anspruch 11, **dadurch gekennzeichnet, dass** die Lutschpastille 100,00 mg Pelargonium-Urtinktur und 25,0 mg Plantago-Trockenextrakt umfasst.

13. Verwendung gemäß Anspruch 11, **dadurch gekennzeichnet, dass** es sich um eine Lutschpastille handelt und dass diese die folgende Zusammensetzung aufweist:
| | |
|---|---|
| Pelargonium-Urtinktur: | 80 - 200 mg |
| Plantago-Trockenextrakt: | 15 - 40 mg |
| Arabisches Gummi: | 300 - 500 mg |
| Maltitollösung: | 400 - 600 mg |
| Sorbitollösung 70 % (nicht kristallisierend): | 100 - 200 mg |
| wasserfreie Citronensäure: | 4 - 9 mg |
| Menthol: | 0 - 100 mg |
| Acesulfam Kalium: | 0,3 - 1 mg |
| Geraniumöl, natürlich: | 0 - 0,1 mg |
| Dünnflüssiges Paraffin: | 0 - 2 mg |
| Gebleichtes Wachs: | 0 - 0,1 mg |
| Prozesswasser: | q.s. |

14. Verwendung gemäß Anspruch 13, **dadurch gekennzeichnet, dass** die Pastille pro Pastille folgendes enthält:
| | |
|---|---|
| Pelargonium-Urtinktur: | 100 mg |
| Plantago-Trockenextrakt: | 25 mg |
| Arabisches Gummi: | 412 mg |
| Maltitollösung: | 553 mg |
| Sorbitollösung 70 % (nicht kristallisierend): | 143 mg |
| wasserfreie Citronensäure: | 6,50 mg |
| Menthol: | 0,80 mg |
| Acesulfam Kalium: | 0,5 mg |
| Geraniumöl, natürlich: | 0,05 mg |
| Dünnflüssiges Paraffin: | 1,30 mg |
| Gebleichtes Wachs: | 0,7 mg |
| Prozesswasser: | q.s., bei einem |
| | Pastillengewicht von 1.000,00 mg. |

## Claims

1. Use of pelargonium in combination with plantago for the manufacture of a pharmaceutical composition in the form of a suckable pastille or suckable tablet for topical prophylaxis and treatment of inflammations of the mouth and pharynx, where pelargonium is *Pelargonium sidoides* and/or *Pelargonium reniforme* and plantago is *Plantago major* and/or *Plantago lanceolata*, where pelargonium is used in the form of an extract.

2. Use according to Claim 1, **characterized in that** initially the effect of the pelargonium ingredient predominates, and subsequently the effect of the plantago ingredient predominates.

3. Use according to Claim 2, **characterized in that** a sequential effect of the ingredients is achieved through the delayed release of plantago.

4. Use according to either of Claims 2 or 3, **characterized in that** the delayed release is achieved by mixing with substances which dissolve at different speeds.

5. Use according to any of the preceding claims, **characterized in that** plantago is also used in the form of an extract.

6. Use according to Claim 5, **characterized in that** the plantago extract is a dry extract.

7. Use according to any of the preceding claims, **characterized in that** the pelargonium extract is a hydroethanolic extract.

8. Use according to any of the preceding claims, involving a bilayer suckable pastille, where an inner core layer is formed by the plantago ingredient, and an outer coating is formed by the pelargonium ingredient.

9. Use according to any of the preceding claims, **characterized in that** a suckable pastille is involved and that the latter comprises as further ingredients gum arabic, maltitol, sorbitol, xylitol, sucrose, isomalt, anhydrous citric acid, acesulfame potassium, low-viscosity paraffin, bleached wax, water, cellulose derivatives, sugar alcohols, Eudragit, and/or aromatizers.

10. Use according to Claim 9, **characterized in that** the aromatizers are geranium oil and/or menthol.

11. Use according to any of the preceding claims, involving a suckable pastille which comprises 80-200 mg of pelargonium mother tincture and 15-40 mg of plantago dry extract.

12. Use according to Claim 11, **characterized in that** the suckable pastille comprises 100.00 mg of pelargonium mother tincture and 25.0 mg of plantago dry extract.

13. Use according to Claim 11, **characterized in that** a suckable pastille is involved, and that the latter has the following composition:
| | |
|---|---|
| Pelargonium mother tincture: | 80-200 mg |
| Plantago dry extract: | 15-40 mg |
| Gum arabic: | 300-500 mg |
| Maltitol solution: | 400-600 mg |
| Sorbitol solution 70% (non-crystallizing): | 100-200 mg |
| Anhydrous citric acid: | 4-9 mg |
| Menthol: | 0-100 mg |
| Acesulfame potassium: | 0.3-1 mg |
| Geranium oil, natural: | 0-0.1 mg |
| Low-viscosity paraffin: | 0-2 mg |
| Bleached wax: | 0-0.1 mg |
| Process water: | q.s. |

14. Use according to Claim 13, **characterized in that** the pastille comprises the following per pastille:
| | |
|---|---|
| Pelargonium mother tincture: | 100 mg |
| Plantago dry extract: | 25 mg |
| Gum arabic: | 412 mg |
| Maltitol solution: | 553 mg |
| Sorbitol solution 70% (non-crystallizing): | 143 mg |
| Anhydrous citric acid: | 6.50 mg |
| Menthol: | 0.80 mg |
| Acesulfame potassium: | 0.5 mg |
| Geranium oil, natural: | 0.05 mg |
| Low-viscosity paraffin: | 1.30 mg |
| Bleached wax: | 0.7 mg |
| Process water: | q.s., for a |
| | pastille weight |
| | of 1000.00 mg. |

## Revendications

1. Utilisation de pélargonium en association avec du plantain pour la fabrication d'une composition pharmaceutique sous forme d'une pastille à sucer ou d'un comprimé à sucer pour la prophylaxie et le traitement topiques d'inflammation de la cavité buccale et pharyngée, le pélargonium étant *Pelargonium sidoides* et/ou *Pelargonium reniforme* et le plantain étant *Plantago major* et/ou *Plantago lanceolata*, le pélargonium étant utilisé sous forme d'un extrait.

2. Utilisation selon la revendication 1**, caractérisée en ce que** d'abord l'action du composant pélargonium prédomine, et à la suite de cela l'action du composant plantain prédomine.

3. Utilisation selon la revendication 2, **caractérisée en ce que** une action décalée dans le temps des composants est causée par la libération retardée du plantain.

4. Utilisation selon la revendication 2 ou 3, **caractérisée en ce que** la libération retardée est provoquée par mélange avec des substances se dissolvant à des vitesses différentes.

5. Utilisation selon l'une quelconque des revendications précédentes, **caractérisée en ce que** le plantain est lui aussi utilisé sous forme d'un extrait.

6. Utilisation selon la revendication 5, **caractérisée en ce que** dans le cas de l'extrait de plantain il s'agit d'un extrait sec.

7. Utilisation selon l'une quelconque des revendications précédentes, **caractérisée en ce que** dans le cas de l'extrait de pélargonium il s'agit d'un extrait aqueux-éthanolique.

8. Utilisation selon l'une quelconque des revendications précédentes, **caractérisée en ce qu'**il s'agit d'une pastille à sucer bicouche, une couche de noyau interne étant constituée du composant plantain et un enrobage externe étant formé par le composant pélargonium.

9. Utilisation selon l'une quelconque des revendications précédentes, **caractérisée en ce qu'**il s'agit d'une pastille à sucer et ce qu'elle comprend comme autres composants de la gomme arabique, du maltitol, du sorbitol, du xylitol, du saccharose, de l'isomalt, de l'acide citrique anhydre, de l'acésulfame potassique, de la paraffine fluide, de la cire blanchie, de l'eau, des dérivés de cellulose, des alcools dérivés de sucres, de l'Eudragit et/ou des arômes.

10. Utilisation selon la revendication 9, **caractérisée en ce que** les arômes consistent en essence de géranium et/ou menthol.

11. Utilisation selon l'une quelconque des revendications précédentes, **caractérisée en ce qu'**il s'agit d'une pastille à sucer qui comprend 80 - 200 mg de teinture-mère de pélargonium et 15 - 40 mg d'extrait sec de plantain.

12. Utilisation selon la revendication 11, **caractérisée en ce que** la pastille à sucer comprend 100,00 mg de teinture-mère de pélargonium et 25,0 mg d'extrait sec de plantain.

13. Utilisation selon la revendication 11, **caractérisée en ce qu'**il s'agit d'une pastille à sucer et **en ce que** celle-ci présente la composition suivante :
| | |
|---|---|
| teinture-mère de pélargonium : | 80-200 mg |
| extrait sec de plantain : | 15-40 mg |
| gomme arabique : | 300-500 mg |
| solution de maltitol : | 400-600 mg |
| solution de sorbitol à 70 % (non cristallisable) : | 100-200 mg |
| acide citrique anhydre : | 4-9 mg |
| menthol : | 0-100 mg |
| acésulfame potassique | 0,3-1 mg |
| essence de géranium, naturelle : | 0-0,1 mg |
| paraffine fluide : | 0-2 mg |
| cire blanchie : | 0-0,1 mg |
| eau de traitement : | q.s. |

14. Utilisation selon la revendication 13, **caractérisée en ce que** la pastille contient, par pastille, ce qui suit :
| | |
|---|---|
| teinture-mère de pélargonium : | 100 mg |
| extrait sec de plantain : | 25 mg |
| gomme arabique : | 412 mg |
| solution de maltitol : | 553 mg |
| solution de sorbitol à 70 % (non cristallisable) : | 143 mg |
| acide citrique anhydre : | 6,50 mg |
| menthol : | 0,80 mg |
| acésulfame potassique | 0,5 mg |
| essence de géranium, naturelle : | 0,05 mg |
| paraffine fluide : | 1,30 mg |
| cire blanchie : | 0,7 mg |
| eau de traitement : | q.s., pour un poids de pastille de 1 000,00 mg |
